(19) Europäisches Patentamt
European Patent Office
Office européen des brevets

(11) Veröffentlichungsnummer: **0 013 414**
**B1**

# (12) EUROPÄISCHE PATENTSCHRIFT

(45) Veröffentlichungstag der Patentschrift: **23.06.82**

(51) Int. Cl.³: **C 07 C 127/22**, C 07 C 149/437, A 01 N 47/34

(21) Anmeldenummer: **79105335.8**

(22) Anmeldetag: **21.12.79**

(54) **Harnstoffderivate, Verfahren zu ihrer Herstellung und ihre Verwendung als Insektizide.**

(30) Priorität: **15.01.79 DE 2901334**
**05.07.79 DE 2927123**

(43) Veröffentlichungstag der Anmeldung: **23.07.80 Patentblatt 80/15**

(45) Bekanntmachung des Hinweises auf die Patenterteilung: **23.06.82 Patentblatt 82/25**

(84) Benannte Vertragsstaaten: **AT BE CH DE FR GB IT LU NL**

(56) Entgegenhaltungen: **DE-A-2 531 202**

(73) Patentinhaber: **CELAMERCK GmbH & Co. KG, Binger Strasse 173, D-6507 Ingelheim am Rhein (DE)**

(72) Erfinder: **Becher, Manfred, Dr., Pfarrer-Heberer-Strasse 5, D-6530 Bingen (DE)**
Erfinder: **Sehring, Richard, Dr., Frankenstrasse 13, D-6507 Ingelheim (DE)**
Erfinder: **Wirtz, Walter, Dr., Reuterallee 12, D-6100 Darmstadt (DE)**
Erfinder: **Prokic-Immel, Ricarda, Dr., Rubensallee 47, D-6500 Mainz (DE)**

**EP 0 013 414 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Harnstoffderivate, Verfahren zu ihrer Herstellung und ihre Verwendung als Insektizide

Die Erfindung betrifft Harnstoffderivate der allgemeinen Formel

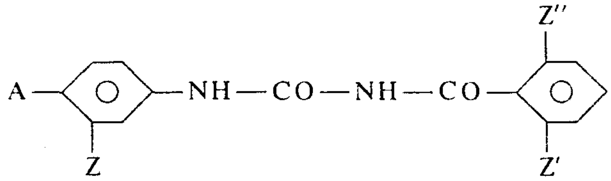

(I)

in der

A für eine der Gruppen

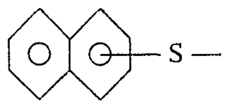

(IIa)

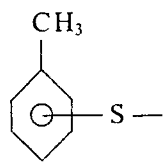

(IIb)

oder

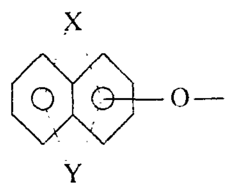

(IIc)

X und Y, die gleich oder verschieden sein können, für Wasserstoff, Chlor oder Brom,

Z für Wasserstoff oder Chlor,

Z' für Chlor oder Fluor und

Z'' für Wasserstoff, Chlor oder Fluor steht

Sie betrifft ferner die Herstellung der Verbindungen der allgemeinen Formel I, ihre Verwendung als Pestizide und pestizide Mittel, welche die neuen Verbindungen als Wirkstoffe enthalten.

Aus der DE-OS 2 531 202 sind insektizide N-Chlorphenyl-N'-dichlorbenzoylharnstoffe bekannt. Es hat sich jedoch gezeigt, daß überraschenderweise die erfindungsgemäßen Benzoylharnstoffe in ihrer Wirkung den bekannten Benzoylharnstoffen deutlich überlegen sind, insbesondere in der Wirkung gegen Epilachna varivestis.

Die Verbindungen der Formel I werden nach an sich bekannten Methoden gewonnen.

1. Durch Umsetzung eines Anilins der Formel

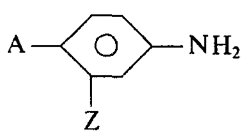

(III)

in der A und Z die obige Bedeutung haben, mit einem Benzolisocyanat der Formel

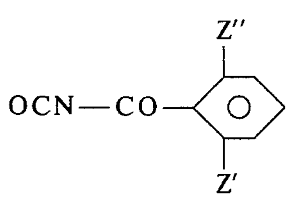

(IV)

in der Z' und Z'' die obige Bedeutung haben.

2. Durch Umsetzung eines Benzamids der Formel

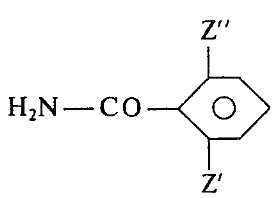

(V)

in derZ' und Z" die obige Bedeutung haben, mit einem Isocyanat der Formel

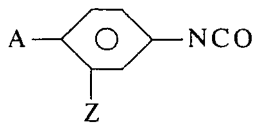

(VI)

in der A und Z die obige Bedeutung haben.

3. Durch Umsetzung eines Anilins der Formel III mit einem Benzoylurethan der Formel

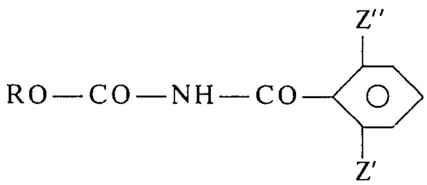

(VII)

in der R einen gegebenenfalls substituierten niederen bis mittleren Alkylrest bedeutet und Z' und Z" die obige Bedeutung haben.

Die Umsetzung gemäß 1. und 2. erfolgt bei Temperaturen zwischen Raumtemperatur und der Siedetemperatur des Reaktionsgemischs in einem inerten Lösungsmittel, z. B. einem aromatischen Kohlenwasserstoff, etwa Toluol oder Xylol, Chlorbenzol, Pyridin, einem Äther wie Dioxan, Tetrahydrofuran, gegebenenfalls in Gegenwart einer tertiären aromatischen Base (Triäthylamin, Pyridin). Es ist vorteilhaft, den Katalysator in Mengen bis etwa 30 Gew.-% des Isocyanats zu verwenden.

Für die Umsetzung der Urethane gemäß 3. wählt man Temperaturen zwischen etwa 60°C und der Siedetemperatur des Reaktionsgemischs. Als Lösungsmittel eignen sich hier vor allem aromatische Kohlenwasserstoffe, etwa Toluol, Xylol, Chlorbenzol.

Die Ausgangsstoffe können nach üblichen Methoden erhalten werden. So können die Aniline der Formel III aus einem Alkalisalz eines $\alpha$- oder $\beta$-Naphthols der Formel

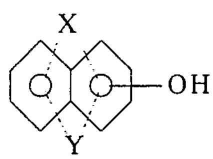

(VIII)

durch Umsetzung mit der Nitroverbindung

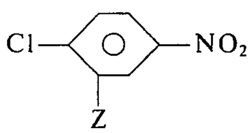

(IX)

und Reduktion des entstandenen Äthers der Formel

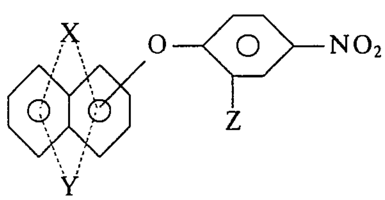

(X)

gewonnen werden. Es ist auch möglich, die Halogenatome in entsprechende Ausgangsstoffe der Formel X (X und/oder Y gleich H) nachträglich einzuführen.

Aus dem Hydrochlorid des Anilins III kann mit Phosgen in üblicher Weise das Isocyanat VI erhalten werden.

Das Urethan der Formel VII ist durch Umsetzung des Benzamids

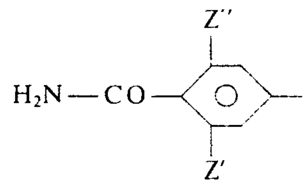

(XI)

mit einem entsprechenden Chlorameisensäureester zugänglich.

Die neuen Verbindungen sind hochwirksame Insektizide. Sie eignen sich besonders zur Bekämpfung von Stechmücken, Raupen, Käfern und Käferlarven.

Hervorzuheben sind diejenigen Verbindungen der Formel I, in denen A für die Teilformel IIc steht und davon vor allem solche, die der Formel

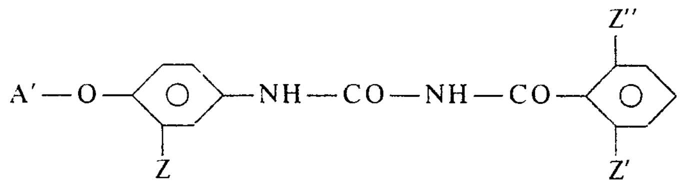

entsprechen und folgende Substituentenkombinationen enthalten:

(1) Für A' gleich Naphthyl(1) oder 4 (bzw. 2)-Chlor-Naphthyl(1)

| Z | / | Z' | / | Z'' |
|---|---|---|---|---|
| H | / | Cl | / | Cl |
| Cl | / | F | / | F |
| H | / | F | / | F |
| H | / | F | / | Cl |
| Cl | / | F | / | Cl |
| H | / | H | / | Cl |

(2) Für A' gleich Naphthyl(2)

| Z | / | Z' | / | Z'' |
|---|---|---|---|---|
| H | / | Cl | / | Cl |
| H | / | F | / | F |
| H | / | F | / | Cl |
| Cl | / | F | / | F |

Für die Anwendung werden die erfindungsgemäßen Wirkstoffe mit üblichen Hilfs- und/oder Trägerstoffen zu gebräuchlichen Formulierungen verarbeitet, z. B. Emulsionskonzentraten, Suspensionspulvern, Stäuben. Die Anwendung erfolgt in Spritzbrühen und Stäuben mit Wirkstoffkonzentrationen zwischen etwa 0,0025 und 2%, in Form von ULV-Formulierungen auch mit höheren Wirkstoffkonzentrationen (bis etwa 90%). Die Aufwandmenge pro Hektar beträgt je nach Wirkstoff und Kultur zwischen etwa 0,05 und 0,5 kg.

Formulierungsbeispiel

Suspensionspulver
(Angaben in Gew.-%)

| | |
|---|---|
| 25% | Wirkstoff gemäß der Erfindung |
| 55% | Kaolin |
| 10% | kolloidale Kieselsäure |
| 9% | Ligninsulfonat (Dispergiermittel) |
| 1% | Natriumtetrapropylenbenzolsulfonat (Netzmittel) |

Die Bestandteile werden wie üblich zu einem Suspensionspulver (Partikelgröße: <4 μ) verarbeitet. Für die Anwendung wird mit Wasser eine Spritzbrühe hergestellt, die etwa 0,0025 bis 0,025 Gew.-% Wirkstoff enthält.

Die überlegene Wirkung der erfindungsgemäßen Verbindungen zeigt sich z. B. im Vergleich des Handelsprodukts der Formel

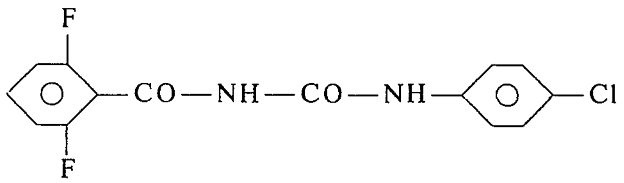
(A)

(Difluobenzuron) mit den erfindungsgemäßen Verbindungen.

Die Werte der nachstehenden Tabelle wurden im Gewächshausversuch ermittelt, wobei die Spritzbrühen aus einer 0,5prozentigen Lösung der Wirkstoffe in Aceton durch Verdünnen mit einer entsprechenden Menge Wasser hergestellt wurden. Als Versuchsobjekte dienten

Aedes aegyptii Larven (4 Tage alt)
Spodoptere littoralis $L_3$ Raupen
Epilachna varivestis $L_3$ Käferlarven.

Die Auswertung erfolgte nach 3 Tagen bei den beiden erstgenannten Schädlingen, nach 6 Tagen bei Epilachna.

Wirkstoffe:
B: N-[4-(α-Naphthoxy)-phenyl]-N'-(2-chlorbenzoyl)-harnstoff
C: N-[4-(α-Naphthoxy)-3-chlorphenyl]-N'-(2,6-di-fluorbenzoyl)-harnstoff
D: N-[4-(β-Naphthoxy)-phenyl]-N'-(2,6-dichlor-benzoyl)-harnstoff
E: N-[4-(α-Naphthoxy)-phenyl]-N'-(2-chlor-6-fluorbenzoyl)-harnstoff
F: N-[4-(β-Naphthoxy)-phenyl]-N'-(2-chlor-6-fluorbenzoyl)-harnstoff
G: N-[4-(α-Naphthoxy)-3-chlorphenyl]-N'-(2-chlor-6-fluorbenzoyl)-harnstoff

| Wirkstoff | A. aegyptii $LD_{95}$ [ppm] | S. littoralis $LD_{95}$ [ppm] | E. varivestis $LD_{95}$ [ppm] |
|---|---|---|---|
| A | 0,0031 | 3,6 | 15,0 |
| B | 0,0044 | 0,38 | – |
| C | 0,00037 | – | 8,8 |
| D | – | 0,9 | 10,5 |
| E | 0,00044 | 0,36 | 7,2 |
| F | 0,0035 | 0,30 | 7,0 |
| G | 0,00094 | 1,7 | 9,0 |

Ein weiterer Vergleich wurde zwischen A und

H: N-(4-Naphthylmercaptophenyl)-N'-(2,6-dichlorbenzoyl)-harnstoff
I: N-(4-Tolylmercaptophenyl)-N'-(2,6-dichlorbenzoyl)-harnstoff

durchgeführt. Gewächshausversuch: 0,5prozentige Lösung der Wirkstoffe in Aceton, mit Wasser verdünnt auf 5 ppm (Versuch 1) bzw. 2 ppm (Versuch 2):

1. Prodenia-Raupen, % Wirkung bei Kontrolle nach 3 Tagen:
   A: 64%
   H: 100%
2. Epilachna-Larven, % Wirkung bei Kontrolle nach 6 Tagen:
   A: 67%
   I: 96%

## Herstellung von Ausgangsstoffen

### A. Nitroverbindungen

#### 1) 4-(α-Naphthoxy)-3-chlor-nitrobenzol

Zu der Lösung von 144 g (1 Mol) α-Naphthol in 1 Ltr. Xylol werden 65 g (1 Mol) fein gepulvertes 88%iges Kaliumhydroxid gegeben. Die so erhaltene Mischung wird unter $N_2$-Atmosphäre und gutem Rühren so lange an einem gut wirksamen Wasserabscheider gekocht, bis sich kein Wasser mehr abscheidet und die Kopftemperatur auf 137° C gestiegen ist. Danach werden 500 ml Dimethylformamid zugesetzt. Die so erhaltene Lösung wird weitere 2 Stunden unter Stickstoff am Wasserabscheider gekocht; anschließend werden von ihr 500 ml Lösungsmittel abdestilliert. Den Rückstand läßt man unter Stickstoff auf ca. 100° C abkühlen und gibt 211 g (1 Mol) 3,4-Dichlornitrobenzol und 1 g Kupferpulver zu. Dann läßt man unter Stickstoff und Rühren 8 Stunden lang am Rückfluß sieden (ca. 140° C Sumpftemperatur). Danach läßt man auf ~50° C abkühlen, gibt ca. 30 g Kieselgur zu, verrührt die so entstandene Mischung, saugt das Ungelöste ab und wäscht es mit Xylol. Das mit der Waschflüssigkeit vereinigte Filtrat wird im Vakuum zur Trockne eingeengt. Der Rückstand wird in warmem Toluol gelöst. Die erhaltene Lösung wird dreimal mit je 500 ml 1 n Natronlauge und anschließend zweimal mit je 500 ml Wasser ausgeschüttelt. Dann wird die Toluolphase im Vakuum zur Trockne eingeengt (Rohausbeute: 290 g, 96% d. Th.). Der Rückstand wird mit 1,7 Ltr, Benzin (Siedebereich 80—110° C) bei ca. 85° C verrührt. Der größte Teil des Rohprodukts geht in Lösung; etwas Harz bleibt ungelöst. Die von ihm abdekantierte Lösung läßt man langsam abkühlen. Das Produkt fällt zunächst ölig aus; durch Verreiben bei ca. 50° C erhält man jedoch Kristalle. Danach kühlt man einige Stunden lang auf 5° C und saugt dann das kristalline Produkt ab.
Ausbeute: 272 g (0,907 Mol), 90,7% d. Th.
Fp. 78—80° C.

#### 2) 4-(α-Naphthoxy)-nitrobenzol

Arbeitsweise wie bei 1). Anstelle des 3,4-Dichlornitrobenzols wird die gleiche molare Menge p-Chlornitrobenzol eingesetzt.
Ausbeute: >90% d. Th.
Fp. 138—140° C.

#### 3) 4-(β-Naphthoxy)-3-chlor-nitrobenzol

Arbeitsweise wie bei 1), jedoch wurde auf die Reinigung durch Behandeln mit Benzin verzichtet. Anstelle des α-Naphthols wurde β-Naphthol eingesetzt.
Ausbeute: prakt. quantitativ

#### 4) 4-(β-Naphthoxy)-nitro-benzol

Arbeitsweise wie bei 3). Anstelle des 3,4-Dichlornitrobenzols wurde die gleiche molare Menge p-Chlornitrobenzol eingesetzt.
Ausbeute: prakt. quantitativ.

5) 4-(1-Chlor-naphthoxy(2))-3-chlor-nitro-benzol

Arbeitsweise wie bei 1). Anstelle des α-Naphthols wird die gleiche molare Menge 1-Chlor-naphthol-(2) eingesetzt. Ausbeute: 79% d. Th., Fp. 117—120° C.

6) 4-(1-Chlor-naphthoxy(2))-nitro-benzol

Arbeitsweise wie bei 1). Anstelle des α-Naphthols wird die gleiche molare Menge 1-Chlor-naphthol-(2) und anstelle des 3,4-Dichlornitrobenzols die gleiche molare Menge p-Chlornitrobenzol eingesetzt.
Ausbeute: 86% d. Th.
Fp. 108—109° C.

7) Herstellung von 4-(Chlornaphthoxy(1))-3-chlor-nitrobenzol durch Chlorieren von 4-(α-Naphthoxy)-3-chlor-nitrobenzol

75 g (0,25 Mol) 4-(α-Naphthoxy)-3-chlor-nitrobenzol werden in 1 Ltr. Eisessig heiß gelöst. Anschließend werden etwas Benzoylperoxid und 40 g (0,30 Mol) Sulfurylchlorid zugefügt. Die so entstandene Mischung wird 4 Stunden lang am Rückfluß gekocht.

Danach wird im Vakuum zur Trockne eingeengt. Der Rückstand wird in Toluol gelöst und die Lösung mehrmals mit Wasser ausgeschüttelt. Anschließend wird die Toluolphase im Vakuum auf Rückstand eingeengt.
Rohausbeute: 80,5 g (0,24 Mol); 96% d. Th.
Umkristallisation des kristallin gewordenen Rohprodukts aus Isopropylalkohol oder aus Essigester.
Ausbeute nach einmaliger Umkristallisation aus Essigester: 54,3 g (0,16 Mol, 65% d. Th.)
Fp. 156—158° C.

8) Herstellung von 4-(Chlornaphthoxy(1))-nitrobenzol durch Chlorieren von 4-(α-Naphthoxy)-nitrobenzol

Arbeitsweise wie bei 7). Anstelle des 4-(α-Naphthoxy)-3-chlor-nitrobenzols wird die gleiche molare Menge 4-(α-Naphthoxy)-nitrobenzol eingesetzt.
Rohausbeute: 97% d. Th.
Umkristallisation des kristallin gewordenen Rohproduktes aus Diisopropyläther.
Ausbeute nach einmaliger Umkristallisation: 67% d. Th.
Fp. 63—64° C.
Wahrscheinlich liegt eine Mischung zweier Isomeren vor (Cl in 2- oder 4-Stellung des Naphthalinrestes).

9) Herstellung von 4-(Chlornaphthoxy(2))-3-chlor-nitrobenzol durch Chlorieren von 4-(β-Naphthoxy)-3-chlor-nitrobenzol

Arbeitsweise wie bei 7). Anstelle des 4-(α-Naphthoxy)-3-chlor-nitrobenzols wird die gleiche Menge 4-(β-Naphthoxy)-3-chlor-nitrobenzol eingesetzt.
Rohausbeute: 97% d. Th.
Umkristallisation des kristallin gewordenen Rohprodukts aus Essigester.
Ausbeute nach einmaliger Umkristallisation: 78% d. Th.
Fp. 120—122° C.
Produkt ist identisch mit dem nach 5) hergestellten.

10) Herstellung von 4-(Chlornaphthoxy(2))-nitrobenzol durch Chlorieren von 4-(β-Naphthoxy)-nitrobenzol

Arbeitsweise wie bei 7). Anstelle des 4-(α-Naphthoxy)-3-chlor-nitrobenzols wird die gleiche molare Menge 4-(β-Naphthoxy)-nitrobenzol eingesetzt.
Rohausbeute: 95% d. Th.
Umkristallisation des kristallin gewordenen Rohprodukts aus Diisopropyläther.
Ausbeute nach einmaliger Umkristallisation: 73% d. Th.
Fp. 111—112° C.
Das Produkt ist identisch mit dem nach 6) hergestellten.

11) Herstellung von 4-(Bromnaphthoxy⟨1⟩)-nitrobenzol durch Bromieren von 4-(α-Naphtoxy)-nitrobenzol

In die 70°C warme Lösung von 26,5 g (0,10 Mol) 4-(α-Naphthoxy)-nitrobenzol in 600 ml Eisessig, der etwas Eisenpulver beigefügt ist, wird unter Rühren die Lösung von 18 g (0,11 Mol) Brom in 50 ml Eisessig zugetropft. Nach beendeter Zugabe rührt man noch ca. 15 Stunden lang bei 25—30°C. Danach engt man die Reaktionslösung im Vakuum zur Trockne ein. Der Rückstand wird in Toluol gelöst. Die so erhaltene Lösung wird mehrfach mit Wasser ausgeschüttelt. Anschließend wird sie im Vakuum zur Trockne eingeengt.
Rohausbeute: 33,5 g (0,097 Mol), 97% d. Th.
Das zunächst ölige Rohprodukt wird langsam kristallin. Es wird dann aus Diisopropyläther umkristallisiert.
Ausbeute nach einmaliger Umkristallisation: 24,5 g (0,71 Mol), 71% d. Th.
Fp. 71—72°C
(Br in 2- oder 4-Stellung des Naphthalinrestes; wahrscheinlich liegt ein Gemisch beider Isomeren vor).

12) Herstellung von 4-(Bromnaphthoxy⟨1⟩)-3-chlor-nitrobenzol durch Bromieren von 4-(α-Naphthoxy)-3-chlor-nitrobenzol

Arbeitsweise wie bei 11). Anstelle des 4-(α-Naphthoxy)-nitrobenzols wird die gleiche molare Menge 4-(α-Naphthoxy)-3-chlor-nitrobenzol eingesetzt.
Rohausbeute: 97% d. Th.
Reinigung durch Aufschlämmen in wenig Diisopropyläther.
Reinausbeute: 66% d. Th.
Fp. 153—155°C.

13) Herstellung von 4-(Bromnaphthoxy⟨2⟩)-nitrobenzol durch Bromieren von 4-(α-Naphthoxy)-nitrobenzol

Arbeitsweise wie bei 11). Anstelle des 4-(α-Naphthoxy)-nitrobenzols wird die gleiche Menge 4-(β-Naphthoxy)-nitrobenzol eingesetzt.
Rohausbeute: 97% d. Th.
Reinigung der heißen Toluol-Lösung des Rohprodukts mit Kieselgel und anschließende Umkristallisation aus Diisopropyläther.
Reinausbeute: 68% d. Th.
Fp. 131—133°C.

14) Herstellung von 4-(Bromnapthoxy⟨2⟩)-3-chlor-nitrobenzol durch Bromieren von 4-(β-Naphthoxy)-3-chlor-nitrobenzol

Arbeitsweise wie bei 11). Anstelle des 4-(α-Naphthoxy)-nitrobenzols wird die gleiche molare Menge 4-(β-Naphthoxy)-3-chlor-nitrobenzol eingesetzt.
Rohausbeute: 96%
Reinigung wie bei 13)
Reinausbeute: 57% d. Th.
Fp. 105—107°C.

15) Herstellung von 4-(2,4-Dichlornaphthoxy⟨1⟩)-3-chlor-nitrobenzol durch Chlorieren von 4-(α-Naphthoxy)-3-chlor-nitrobenzol

In die 50°C warme Lösung von 30 g (0,10 Mol) 4-(α-Naphthoxy)-3-chlor-nitrobenzol und 8,2 g (0,10 Mol) Natriumacetat wird nach Zugabe von 0,5 g Eisen(III)-Chlorid unter Rühren so lange Chlor eingeleitet, bis die Reaktionsmischung 14,2 g (0,10 Mol) Chlor aufgenommen hat. Danach wird noch ca. 15 Minuten lang bei 50°C gerührt. Anschließend wird das Ungelöste abgesaugt und das Filtrat im Vakuum auf Rückstand eingeengt, der in Diisopropyläther gelöst wird. Diese Lösung wird mehrfach mit Wasser ausgeschüttelt, danach mit Magnesiumsulfat getrocknet und zuletzt auf Rückstand eingeengt. Der so behandelte Rückstand wird in kochendem Benzin (Siedebereich 80—110°C) gelöst. Beim Abkühlen fällt zunächst ein Harz aus, von dem die Benzinlösung dekantiert wird. Aus ihr kristallisiert das Produkt langsam aus. Es wird abgesaugt und mit Benzin nachgewaschen.
Ausbeute: 12,4 g (0,041 Mol), 41% d. Th.
Fp. 121—123°C.

Alle gereinigten Nitroverbindungen wurden analysiert. Die C-, H-, N- und gegebenenfalls Halogen-Werte stimmen in allen Fällen mit den theoretischen Werten überein. Außerdem wurden die NMR-Spektren dieser gereinigten Produkte aufgenommen. Auch sie passen zu den angegebenen Strukturen.

## B. Aniline

### 1) 4-(α-Naphthoxy)-anilin

Die Mischung von 1,5 Ltr. Wasser, 40 ml Eisessig und 1,0 Mol 4-(α-Napthoxy)nitrobenzol wird unter Rühren zum Sieden erhitzt. Nach Erreichen der Siedetemperatur werden 200 g Eisenpulver in kleinen Portionen eingetragen. Nach beendeter Zugabe wird 6 Stunden lang unter Rühren am Rückfluß gekocht.

Danach läßt man das Reaktionsgemisch auf Raumtemperatur abkühlen. Dabei setzen sich die in Wasser ungelösten Bestandteile ab. Nach einigen Stunden wird die überstehende klare wäßrige Phase vorsichtig dekantiert. Zu dem zurückbleibenden Gemisch gibt man 1,5 Ltr. Aceton, so daß sich die organischen Bestandteile lösen. Das ungelöst bleibende wird abgesaugt und mit Aceton nachgewaschen. Nach Vereinigung der Filtrate wird das Aceton größtenteils abdestilliert. Die Destillation wird abgebrochen, wenn die Dampftemperatur bei 95°C liegt. Den heterogenen Rückstand verrührt man nach dem Abkühlen mit 1 Ltr. Äthylenchlorid so lange, bis nur noch zwei flüssige Phasen vorliegen. Sie werden getrennt und die wäßrige nochmals mit 200 ml Äthylenchlorid extrahiert. Die organischen Phasen werden vereinigt, mit Magnesiumsulfat getrocknet und auf Rückstand eingeengt. Zurück bleibt öliges Rohprodukt, das an der Ölpumpe destilliert wird. Der Hauptlauf wird langsam kristallin.

Rohausbeute: 220 g (0,936 Mol); 93% d. Th.
Reinausbeute: 195 g (0,830 Mol); 83% d. Th.
Sdp.: 181—186°C/0,6—0,7 mbar
Fp.: 49—50°C

### 2) 4-(β-Naphthoxy)-anilin

Zur Reduktion des 4-(β-Naphthoxy)-nitrobenzols verfährt man wie unter 1) (1/4 des dort beschriebenen Ansatzes). Bei der Destillation des Rohprodukts geht als Vorlauf etwas Hexametapol und p-Chloranilin über. Der Hauptlauf wird kristallin.

Reinausbeute:
41 g (0,174 Mol)
70% d. Th. über beide Stufen
Sdp.: 194—196°C/0,7 mbar
Fp.: 113—115°C.

### 3) 4-(α-Naphthoxy)-3-chloranilin

Man arbeitet analog zu 1), ausgehend von 4-(α-Naphthoxy)-3-chlor-nitrobenzol (1/4 des dort beschriebenen Ansatzes). Bei der Destillation des Rohprodukts geht als Vorlauf etwas Hexametapol und 3,4-Dichloranilin über. Der Hauptlauf bleibt auch nach längerer Zeit hochviskos ölig.

Reinausbeute:
50,4 g (0,187 Mol)
75% d. Th. über beide Stufen
Sdp.: 193—197°C/0,3 mbar

Weitere analog hergestellte Aniline:

(a) 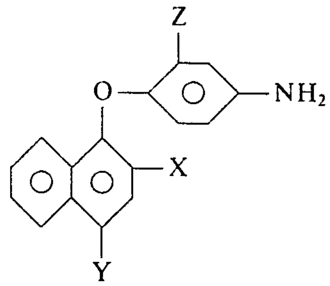

| Nr. | X | Y | Z | Fp. | Kp. |
|---|---|---|---|---|---|
| 4*) | H | Cl | H | | |
| 5*) | H | Cl | Cl | 98–100°C | |
| 6*) | H | Br | H | | |
| 7*) | H | Br | Cl | 92–94°C | |
| 8 | Cl | Cl | Cl | | |

*) Möglicherweise enthalten die Produkte z. T. die entsprechenden Isomeren mit X gleich Halogen und Y gleich H.

(b) 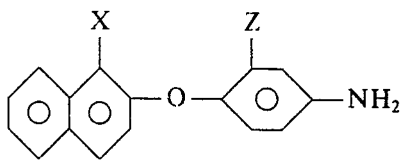

| Nr. | X | Z | Fp. | Kp. |
|---|---|---|---|---|
| 9 | H | Cl | 70–71°C | ca. 195°C/ 0,13, bar |
| 10 | Cl | H | 91–93°C | |
| 11 | Cl | Cl | 90–92°C | |
| 12 | Br | H | 94–96°C | |
| 13 | Br | Cl | 100–102°C | |

C. Endprodukte der Formel

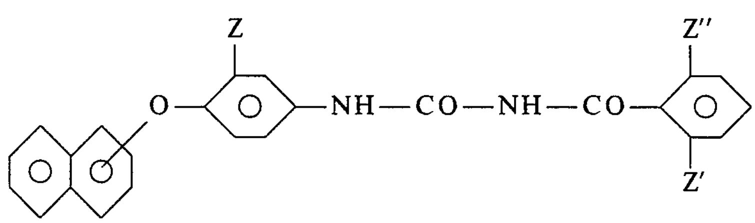

**Beispiel 1**

**N-[4-(α-Naphthoxy)-phenyl]-N'-(2-chlor-benzoyl)-harnstoff**

Zu der Lösung von 4,7 g (0,020 Mol) 4-(α-Naphthoxy)-anilin in 100 ml absolutem Toluol werden 3,65 g (0,020 Mol) O-Chlorbenzoylisocyanat gegeben. Die so erhaltene Reaktionslösung wird etwa

15 Stunden lang bei Raumtemperatur gerührt. In dieser Zeit kristallisiert das Produkt aus. Es wird abgesaugt, mit Toluol nachgewaschen und getrocknet.
Ausbeute: 7,9 g (0,019 Mol), 95% d. Th.
Fp. 219—221°C.

Die anschließend genannten Harnstoff-Derivate werden in gleicher Weise unter Einsatz gleicher molarer Mengen der entsprechenden Naphthoxyaniline und der entsprechend substituierten Benzoylisocyanate dargestellt. Dabei werden Ausbeuten von 85—95% d. Th. erzielt.

(a) N-[4-(α-Naphthoxy)-3-chlor-phenyl]-N'-(2-chlor-benzoyl)-harnstoff
Fp.: 221—223°C.
(b) N-[4-(α-Napthoxy)-phenyl]-N'-(2,6-dichlor-benzoyl)-harnstoff
Fp.: 205—208°C.
(c) N-[4-(β-Naphthoxy)-phenyl]-N'-(2,6-dichlor-benzoyl)-harnstoff
Fp.: 217—220°C.
(d) N-[4-(α-Naphthoxy)-phenyl]-N'-(2,6-difluor-benzoyl)-harnstoff
Fp.: 215—218°C.
(e) N-[4-(β-Naphthoxy)-phenyl]-N'-(2,6-difluor-benzoyl)-harnstoff
Fp.: 219—222°C.
(f) N-[4-(α-Naphthoxy)-3-chlor-phenyl]-N'-(2,6-difluor-benzoyl)-harnstoff
Fp.: 229—232°C.
(g) N-[4-(α-Naphthoxy)-phenyl]-N'-(2-chlor-6-fluor-benzoyl)-harnstoff
Fp.: 209—211°C.
(h) N-[4-(β-Naphthoxy)-phenyl]-N'-(2-chlor-6-fluor-benzoyl)-harnstoff
Fp.: 206—208°C.
(i) N-[4-(α-Naphthoxy)-3-chlor-phenyl]-N'-(2-chlor-6-fluor-benzoyl)-harnstoff
Fp.: 242—245°C.

## Beispiel 2

### N-[4-(α-Naphthoxy)-phenyl]-N'-(2,6-dichlor-benzoyl)-harnstoff

### 1. Stufe

### Herstellung des Isocyanats

In die Lösung von 23,5 g (0,10 Mol) 4-(α-Naphthoxy)-anilin in 200 ml Dioxan wird unter Rühren bei Raumtemperatur so lange Chlorwasserstoff eingeleitet, bis kein Hydrochlorid mehr ausfällt. Nach Abkühlen der so entstandenen Mischung auf 5—10°C werden in sie noch 15 g Phosgen unter intensivem Rühren eingeleitet. Nach Beendigung des Einleitens rührt man 3 Stunden lang bei 25°C, anschließend 1 Stunde bei 50°C und zuletzt noch eine Stunde bei 95—100°C. Dabei entsteht eine klare Lösung. Dann wird überschüssiges Phosgen bei ca. 95°C durch Einleiten von Stickstoff abgetrieben. Anschließend wird das Lösungsmittel im Vakuum abdestilliert. Das ölige Produkt bleibt zurück.
Ausbeute:
26 g (0,995 Mol) 4-(α-Naphthoxy)-phenyl-isocyanat
(prakt. quantitative Ausbeute)

### 2. Stufe

### Herstellung des Harnstoffes

Zu der Lösung von 13,1 g (0,050 Mol) 4-(α-Naphthoxy)-phenylisocyanat in 100 ml Dioxan werden 9,5 g (0,50 Mol) 2,6-Dichlorbenzoylamid und 2 ml Triäthylamin gegeben. Die so entstandene Mischung wird unter Rühren 15 Stunden lang am Rückfluß gekocht. Danach wird das Lösungsmittel abdestilliert, der Rückstand wird mit 100 ml Toluol verrieben. Das Produkt bleibt ungelöst kristallin. Es wird abgesaugt und getrocknet.
Ausbeute: 19,4 g (86% d. Th.)
Fp.: 194—198°C

Die 2. Stufe kann auch wie folgt durchgeführt werden:

Zu der Lösung von 13,1 g (0,05 Mol) 4-(α-Naphthoxy)-phenylisocyanat und 9,5 g (0,050 Mol) 2,6-Dichlorbenzoylamid in 50 ml Pyridin wird 1 g Natrium zugesetzt. Das so erhaltene Gemisch wird 24 Stunden lang auf 100°C erhitzt. Danach läßt man es auf Raumtemperatur abkühlen und rührt das abgekühlte Reaktionsgemisch in eine Mischung von 250 g Eis und 250 ml konz. Salzsäure ein. Der dabei auskristallisierende Niederschlag wird abgesaugt und zunächst mit Wasser, danach mit Äthanol

gewaschen und getrocknet.
Ausbeute: 18,8 g (0,0417 Mol); 83% d. Th.
Fp.: 196—200°C.

Analog erhält man:

N-[4-(α-Naphthoxy)-phenyl]-N'-(2-chlorbenzoyl)-harnstoff
Fp.: 211—215°C,
N-[4-(α-Naphthoxy)-3-chlor-phenyl]-N'-(2-chlorbenzoyl)-harnstoff
Fp.: 214—218°C,
N-[4-(β-Naphthoxy)-phenyl]-N'-(2,6-dichlor-benzoyl)-harnstoff
Fp.: 210—214°C,
N-[4-(α-Naphthoxy)-phenyl]-N'-(2,6-difluor-benzoyl)-harnstoff
Fp.: 211—214°C,
N-[4-(β-Naphthoxy)-phenyl]-N'-(2,6-difluor-benzoyl)-harnstoff
Fp.: 213—217°C,
N-[4-(α-Naphthoxy)-3-chlor-phenyl]-N'-(2,6-difluor-benzoyl)-harnstoff
Fp.: 222—226°C,
N-[4-(α-Naphthoxy)-phenyl]-N'-(2-chlor-6-fluor-benzoyl)-harnstoff
Fp.: 205—208°C,
N-[4-(β-Naphthoxy)-phenyl]-N'-(2-chlor-6-fluor-benzoyl)-harnstoff
Fp.: 201—204°C,
N-[4-(α-Naphthoxy)-3-chlor-phenyl]-N'-(2-chlor-6-fluor-benzoyl)-harnstoff
Fp.: 235—239°C.

## Beispiel 3

N-[4-(α-Naphthoxy)-phenyl]-N'-(2,6-dichlorbenzoyl)-harnstoff

Das N-(2,6-Dichlor-benzoyl)-O-äthylurethan ist durch Umsetzen einer Suspension des Natriumsalzes von 2,6-Dichlor-benzamid in Dioxan mit Chlorameisensäureäthylester zugänglich. Durch Behandeln des Rohprodukts mit Toluol oder Xylol kann nicht umgesetztes 2,6-Dichlorbenzamid abgetrennt werden, da das Urethan in diesen Lösungsmitteln gut löslich, das Amid jedoch nur schwer löslich ist.

Die Lösung von 4,70 g (0,020 Mol) 4-(α-Naphthoxy)-anilin und 5,75 g (0,022 Mol) N-(2,6-Dichlorbenzoyl)-O-äthylurethan in 60 ml absolutem Xylol wird in einer Destillationsapparatur mit kurzer Füllkörperkolonne und einem Tropftrichter zum Nachgeben von Xylol in dem Maß erhitzt, daß der entstehende Alkohol und das Lösungsmittel sehr langsam übergehen. Während dieser Destillation wird zum Reaktiongemisch soviel Xylol getropft, wie Destillat übergeht. Die Kopftemperatur steigt von zunächst etwa 80°C auf etwa 137°C. Nach 5stündigem Destillieren wird die Lösungsmittelzugabe beendet und das Lösungsmittel des Reaktionsgemisches ohne Kolonne abdestilliert, zuletzt im Vakuum. Zurück bleiben 9,6 g eines Substanzgemischs, dessen Hauptbestandteil der N-[4-(α-Naphthoxy)-phenyl]-N'-(2,6-dichlorbenzoyl)-harnstoff ist.

## D. Endprodukte der Formel

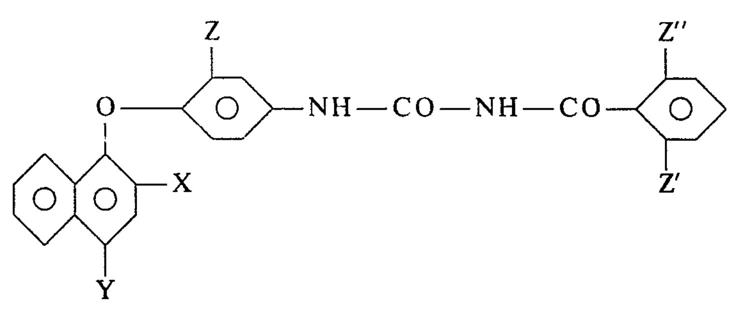

Die Verbindungen in der folgenden Liste werden analog den unter C aufgeführten Beispielen erhalten.

| Nr. | Y | X | Z | Z' | Z" | F. [°C] |
|---|---|---|---|---|---|---|
| 1 | Cl | H | H | Cl | Cl | 240–242 |
| 2 | Cl | H | H | F | F | 213–215 |
| 3 | Cl | H | H | Cl | F | 220–222 |
| 4 | Cl | H | H | Cl | H | 221–223 |
| 5 | Cl | H | Cl | Cl | Cl | 250–252 |
| 6 | Cl | H | Cl | F | F | 232–234 |
| 7 | Cl | H | Cl | Cl | F | 246–248 |
| 8 | Cl | H | Cl | Cl | H | 214–216 |
| 9 | Br | H | H | Cl | Cl | 245–247 |
| 10 | Br | H | H | F | F | 214–215 |
| 11 | Br | H | H | Cl | F | 227–229 |
| 12 | Br | H | H | Cl | H | 216–218 |
| 13 | Br | H | Cl | Cl | Cl | 245–247 |
| 14 | Br | H | Cl | F | F | 228–230 |
| 15 | Br | H | Cl | Cl | F | 233–236 |
| 16 | Br | H | Cl | Cl | H | 219–222 |
| 17 | Cl | Cl | Cl | Cl | Cl | 247–249 |
| 18 | Cl | Cl | Cl | F | F | 255–257 |
| 19 | Cl | Cl | Cl | Cl | F | 251–253 |
| 20 | Cl | Cl | Cl | Cl | H | 242–245 |

Anmerkung zu Verbindung Nr. 1–16: Möglicherweise enthalten die Produkte z. T. die Isomeren mit Y gleich Wasserstoff und X gleich Halogen.

E. Endprodukte der Formel

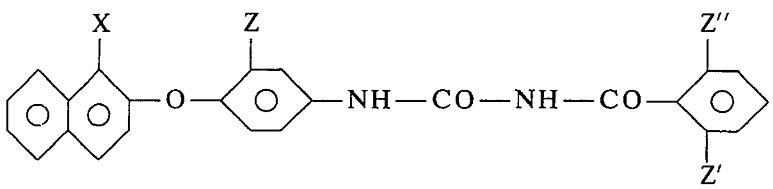

Die Verbindungen der folgenden Liste werden analog den unter C aufgeführten Beispielen erhalten:

| Nr. | X | Z | Z' | Z" | Fp. [°C] |
|---|---|---|---|---|---|
| 1 | Cl | H | Cl | Cl | 227–229 |
| 2 | Cl | H | F | F | 237–239 |
| 3 | Cl | H | Cl | F | 235–238 |
| 4 | Cl | H | Cl | H | 227–229 |
| 5 | Cl | Cl | Cl | Cl | 253–255 |
| 6 | Cl | Cl | F | F | 244–246 |
| 7 | Cl | Cl | Cl | F | 255–257 |
| 8 | Cl | Cl | Cl | H | 230–232 |
| 9 | Br | H | Cl | Cl | 235–237 |
| 10 | Br | H | F | F | 234–236 |
| 11 | Br | H | Cl | F | 233–235 |
| 12 | Br | H | Cl | H | 225–227 |
| 13 | Br | Cl | Cl | Cl | 251–255 |
| 14 | Br | Cl | F | F | 243–245 |
| 15 | Br | Cl | Cl | F | 246–248 |
| 16 | Br | Cl | Cl | H | 230–233 |

F. Verbindungen der Formel I, in denen A die Gruppe IIa oder IIb bedeutet

Beispiel 4

N-(4-Naphthylmercapto-phenyl)-N'-(2,6-dichlorbenzoyl)-harnstoff

a) p-Nitrobenzolsulfenylchlorid

Zu der Aufschlämmung von 30,8 g (0,10 Mol) Di-(4-nitrophenyl)-disulfid in 200 ml Chloroform wird unter Rühren bei 25–30° C Innentemperatur so lange Chlor eingeleitet, bis eine klare Lösung vorliegt und kein Chlor mehr aufgenommen wird. Danach wird noch 3 Stunden lang bei 40–45° C gerührt. anschließend destilliert man das Lösungsmittel im Vakuum bei maximal 50° C Badtemperatur ab. Öliges 4-Nitrobenzolsulfenylchlorid bleibt zurück.

b) 4-Nitrophenyl-naphthyl-thioäther

Das rohe 4-Nitrobenzolsulfenylchlorid (38 g Produkt der vorhergehenden Stufe) wird bei 80° C zu 200 g geschmolzenem Naphthalin gegeben. Anschließend werden noch 0,5 g Fe(III)-chlorid eingetragen. Danach wird unter Rühren so hoch erhitzt, daß deutlich Chlorwasserstoff frei wird (in diesem Fall auf ~95° C). Nach Abklingen der Gasentwicklung wird noch 1–2 Stunden lang bei ca. 105° C gerührt.

Danach destilliert man das überschüssige Naphthalin im Wasserstrahlvakuum ab. Der Destillationsrückstand wird in 300 ml Äthylenchlorid gelöst und diese Lösung zweimal mit Wasser ausgeschüttelt. Die organische Phase wird nach Zugabe von 0,5 l Wasser im Vakuum solange destilliert, bis das Destillat klar bleibt. Der Rückstand wird erneut in 300 ml Äthylenchlorid gelöst; das noch vorhandene Wasser wird abgetrennt. Danach wird das Lösungsmittel im Vakuum abdestilliert. Anschließend wird der Rückstand mit 1000 ml Benzin (Siedebereich: 40–80° C) unter Rühren aufgekocht. Danach werden ungelöst vorliegende, feste Bestandteile abfiltriert und noch zweimal mit

je 1000 ml Benzin behandelt. Schließlich bleibt als Nebenprodukt entstandenes Di-(4-nitrophenyl)-disulfid ungelöst, das durch Filtration isoliert wird. Die Filtrate werden vereinigt und auf Rückstand eingeengt. Zurück bleibt das ölige Produkt.
Ausbeute:
31,3 g (0,111 Mol) 4-Nitrophenyl-naphthylthioäther, entsprechend
55,5% d. Th. bez. auf in Stufe a) eingesetztes Di-(4-nitrophenyl)-disulfid
zurückgewonnen:
12,5 g (0,040 Mol) Di-(4-nitrophenyl)-disulfid, entsprechend
~40% der in Stufe a) eingesetzten Menge.

c) 4-Naphthylmercapto-anilin

Zu der Mischung von 31,0 g (0,11 Mol) 4-Nitrophenylnaphthylthioäther (Produkt der vorhergehenden Stufe), 500 ml Wasser und 2 ml Essigsäure werden unter Rühren 34 g Eisenpulver gegeben. Anschließend wird das so erhaltene Gemisch 5 Stunden lang bei 95—100° C gut gerührt. Dabei färbt es sich schwarzbraun.

Danach läßt man das Reaktionsgemisch auf ~60° C abkühlen. Wenn diese Temperatur erreicht ist, gibt man 200 ml Äthylenchlorid und 10 g Kieselgur zu. Anschließend rührt man das so entstandene Gemisch 5 Minuten lang sehr intensiv. Dann saugt man die festen Bestandteile ab. Das Filtrat teilt sich in 2 Phasen auf. Die organische Phase wird abgetrennt, einmal mit 50 ml Wasser ausgeschüttelt und dann im Vakuum auf Rückstand eingeengt. Das ölige Produkt bleibt zurück.
Ausbeute:
25,7 g (0,102 Mol) 4-Naphthylmercapto-anilin, entsprechend
93% d. Th. bez. auf die einges. Nitroverbindung.

d) N-(4-Naphthylmercapto-phenyl)-N'-(2,6-dichlorbenzoyl)-harnstoff

Zu der Lösung von 2,51 g (10 mMol) 4-Naphthylmercapto)-anilin in 20 ml Toluol wird bei Raumtemperatur die Lösung von 2,4 g (11 mMol) 2,6-Dichlorbenzoylisocyanat in 10 ml Toluol gegeben. Dabei erwärmt sich das Reaktionsgemisch auf ~30° C. Es bleibt dann 15 Stunden bei etwa Raumtemperatur stehen. In dieser Zeit kristallisiert ein Teil des Produkts aus. Das auskristallisierte Produkt wird abgesaugt und getrocknet. Das Filtrat wird im Vakuum auf Rückstand eingeengt.
Ausbeute an auskristallisiertem Produkt: 3,3 g, 71% d. Th.
Fp. 207—209° C.

Nach den Ergebnissen der Elementaranalyse und der NMR-Aufnahme liegt das gewünschte Produkt rein vor (bzw. als Isomerengemisch von $\alpha$- und $\beta$-Naphthyl-Verbindung).

Aus der Mutterlauge werden 1,2 g Rückstand (Rohprodukt) erhalten;
Fp. 166—170° C.

Nach Elementaranalyse und NMR-Aufnahme besteht auch diese Substanz vorwiegend aus dem gewünschten Produkt.

Als Alternative zu d) sind folgende Reaktionsschritte möglich:

e) 4-Naphthylmercapto-phenyl-isocyanat

In die Lösung von 25,1 g (0,10 mol) 4-Naphthylmercaptoanilin (Produkt der vorhergehenden Stufe) in 200 ml Dioxan wird unter Rühren bei Raumtemperatur so lange Chlorwasserstoff eingeleitet, bis kein Hydrochlorid mehr ausfällt. Anschließend werden in die so entstandene Mischung nach Abkühlen auf 5—10° C unter intensivem Rühren 15 g Phosgen eingeleitet. Nach Beendigung des Einleitens erwärmt man das Reaktionsgemisch auf etwa 25° C und rührt dann 3 Stunden lang bei dieser Temperatur. In dieser Zeit entsteht eine klare Lösung. Diese wird 1 Stunde bei 50° C und anschließend noch 1 Stunde bei 95—100° C gerührt. Danach wird überschüssiges Phosgen bei ~95° C durch Einleiten von Stickstoff abgetrieben. Anschließend wird das Lösungsmittel im Vakuum abdestilliert. Das ölige Produkt bleibt zurück.
Ausbeute:
27,5 g (0,993 Mol) 4-Naphthylmercapto-phenylisocyanat,
prakt. quantitative Ausbeute.

f) N-(4-Naphthylmercapto-phenyl)-N'-(2,6-dichlorbenzoyl)-harnstoff

Zu der Lösung von 11,0 g (40 mMol) Isocyanat (Produkt der vorhergehenden Stufe) in 100 ml Dioxan werden 7,6 g (40 mMol) 2,6-Dichlorbenzoylamid und 2 ml Triäthylamin zugegeben. Die so entstandene Mischung wird unter Rühren 15 Stunden lang am Rückfluß gekocht. Danach wird das Lösungsmittel im

Vakuum abdestilliert und der Rückstand in einer Mischung von 100 ml Äthylenchlorid und 100 ml Wasser gelöst. Die wäßrige Phase wird dann abgetrennt und die organische noch zweimal mit je 100 ml Wasser ausgeschüttelt. Anschließend wird sie mit Magnesiumsulfat getrocknet. Danach wird das Lösungsmittel im Vakuum abdestilliert. Der zunächst ölige Rückstand wird nach Verreiben mit Wasser kristallin. Die Kristalle werden abgesaugt und im Vakuumexsikkator über konz. Schwefelsäure getrocknet.

Ausbeute:

17,8 g (38,2 mMol) N-(4-Naphthylmercaptophenyl)-N'-(2,6-dichlorbenzoyl)-harnstoff, entsprechend 95% d. Th., bez. auf einges. Isocyanat.

Fp.: 188 – 190° C.

## Beispiel 5

### N-(4-Tolylmercapto-phenyl)-N'-(2,6-dichlorbenzyl)-harnstoff

#### a) 4-Nitrophenyl-tolyl-thioäther

Rohes p-Nitrobenzolsulfenylchlorid (38 g) wird in 200 ml Toluol gelöst. Die Lösung wird nach Zusatz von 0,5 g Eisen(III)-chlorid unter Rühren so hoch erhitzt, daß deutlich Chlorwasserstoff frei wird (in diesem Fall auf ~70 – 80° C). Nach Abklingen der Gasentwicklung wird noch 1 – 2 Stunden lang bei einer um etwa 10° C höheren Temperatur gerührt. Danach läßt man die Lösung auf ~30° C abkühlen und schüttelt sie dreimal mit je 100 ml Wasser aus. Dann wird die organische Phase im Vakuum auf Rückstand eingeengt. Dieser Rückstand wird mit 500 ml Benzin (Siedebereich: 40 – 80° C) aufgekocht. Danach ungelöst vorliegende, feste Bestandteile werden abfiltriert und noch zweimal mit je 500 ml Benzin behandelt. Ungelöst bleibt als Nebenprodukt entstandenes Di-(4-nitrophenyl)-disulfid. Die Filtrate werden vereinigt und auf Rückstand eingeengt. Zurück bleibt das ölige Produkt.

Ausbeute:

25,0 g (0,102 Mol) 4-Nitrophenyl-tolylthioäther;

zurückgewonnen:

13 g (0,042 Mol) Di-(4-nitrophenyl)-disulfid.

#### b) 4-Tolylmercaptoanilin

Ansatz:

24,5 g (0,10 Mol) p-Nitrophenyl-tolylthioäther (Produkt der vorhergehenden Stufe)

Arbeitsweise wie bei 4c).

Ausbeute:

20,4 g (0,095 Mol) 4-Tolylmercapto-anilin, entsprechend 95% d. Th. (bezogen auf eingesetzte Nitroverbindung).

#### c) N-(4-Tolylmercapto-phenyl)-N'-(2,6-dichlorbenzoyl)-harnstoff

Ansatz:

2,15 g (10 mMol) 4-Tolylmercapto-anilin (Produkt der vorhergehenden Stufe)

Arbeitsweise wie bei 4d).

Ausbeute an auskristallisiertem Produkt:

2,13 g (4,9 mMol; 49% d. Th.)

Fp. 189 – 203° C.

Nach den Ergebnissen der Elementaranalyse und der NMR-Aufnahme liegt reines Isomerengemisch vor (Stellung der Methylgruppe des Tolylrestes unsicher).

Ausbeute an Rohprodukt aus der Mutterlauge 2,0 g.

Fp. 148 – 152° C.

Auch dieses Produkt ist nach den Ergebnissen der Elementaranalyse und der NMR-Aufnahme weitgehend reines Isomerengemisch.

Analog erhält man

N-(4-Tolylmercapto-3-chlorphenyl)-N'-(2,6-dichlorbenzoylharnstoff, Fp. 192 – 195° C

Tabelle I

Formel

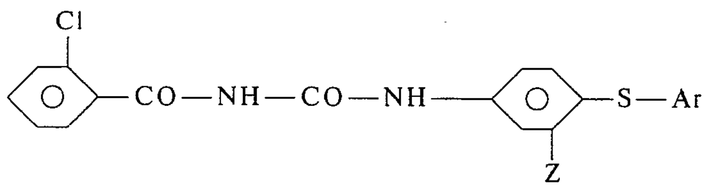

| Beispiel | Z | Ar | Fp. |
|---|---|---|---|
| 6 | H | 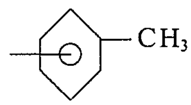 | 170–172°C |
| 7 | Cl | 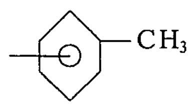 | 186–189°C |
| 8 | Cl | 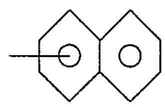 | 227–230°C |

Tabelle II

Formel

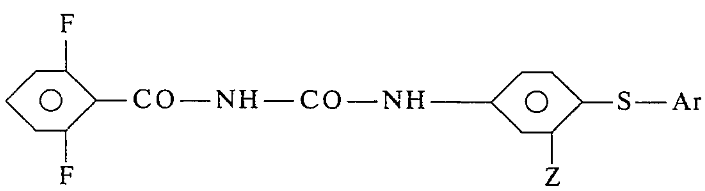

| Beispiel | Z | Ar | Fp. |
|---|---|---|---|
| 9 | H | 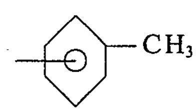 | 186–188°C |
| 10 | H | 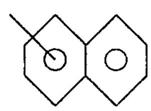 | 213–215°C |
| 11 | Cl | 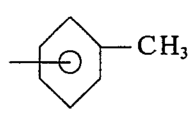 | 197–199°C |
| 12 | Cl | 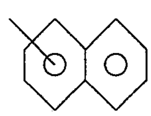 | 236–239°C |

Tabelle III

Formel

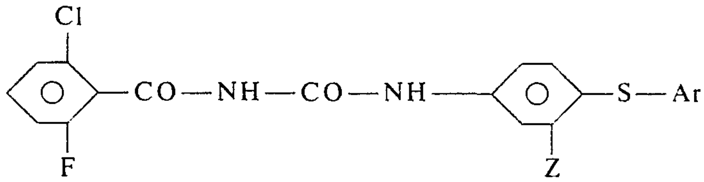

| Beispiel | Z | Ar | Fp. |
|---|---|---|---|
| 13 | H | 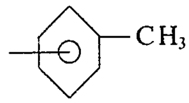 | 138–139°C |
| 14 | H | 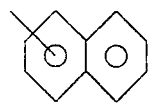 | 197–198°C |
| 15 | Cl | 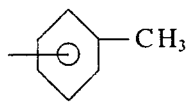 | 201–204°C |
| 16 | Cl | 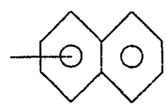 | 245–247°C |

Sämtliche in den Tabellen I–III genannten Verbindungen sind durch Elementaranalysen und NMR-Aufnahmen charakterisiert.

Die als Ausgangsstoffe benutzten Nitroverbindungen der Formel

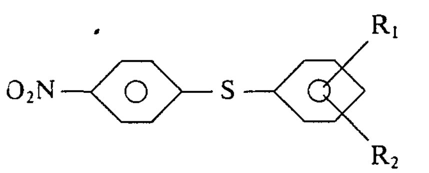

sind in der folgenden Tabelle zusammengestellt:

| Nr. | $R_1$ | $R_2$ | Stellung von $R_1$ und $R_2$ zueinander | Fp. oder Konsistenz der Rohprodukte |
|---|---|---|---|---|
| 1*) | $-H$ | $-CH_3$ | – | Öl |
| 2*) | $-CH=CH-CH=CH-$ | | ○ | Öl |

*) Isomerengemisch hinsichtlich der Stellung von $R_1/R_2$ zu S.

Ausgangsstoffe der Formel

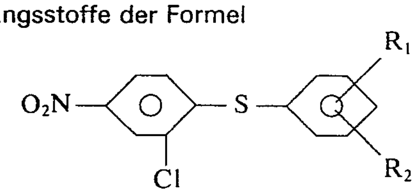

| Nr. | $R_1$ | $R_2$ | Stellung von $R_1$ und $R_2$ zueinander | Fp. oder Konsistenz der Rohprodukte |
|---|---|---|---|---|
| 1*) | $-H$ | $-CH_3$ | – | Öl |
| 2*) | $-CH=CH-CH=CH-$ | | o | Fp. 81–83°C |

*) Isomerengemisch hinsichtlich der Stellung von $R_1/R_2$ zu S.

## Patentansprüche

1. Verbindungen der allgemeinen Formel

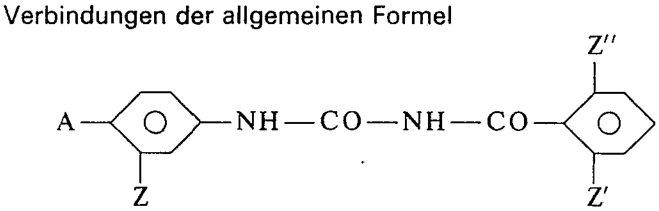

(I)

in der A für eine der Gruppen

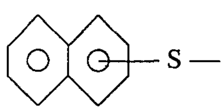

(IIa)

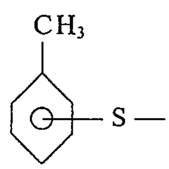

(IIb)

oder

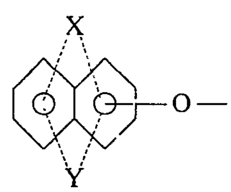

(IIc)

X und Y, die gleich oder verschieden sein können, für Wasserstoff, Chlor oder Brom,
Z für Wasserstoff oder Chlor,
Z' für Chlor oder Fluor und
Z'' für Wasserstoff, Chlor oder Fluor steht.

2. N-[4-($\alpha$-Naphthoxy)-phenyl]-N'-(2-chlorbenzoyl)-harnstoff.
3. N-[4-($\alpha$-Naphthoxy)-phenyl]-N'-(2-chlor-6-fluorbenzoyl)-harnstoff.
4. N-[4-($\beta$-Naphthoxy)-phenyl]-N'-(2-chlor-6-fluorbenzoyl)-harnstoff.
5. Verfahren zur Herstellung von Verbindungen nach Anspruch 1, dadurch gekennzeichnet, daß man

a) ein Anilin der Formel

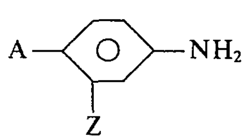

(III)

mit einem Benzoylisocyanat der Formel

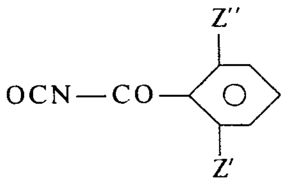

(IV)

umsetzt oder daß man

b) ein Benzamid der Formel

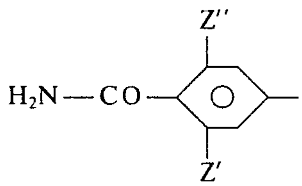

(V)

mit einem Isocyanat der Formel

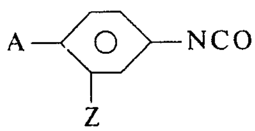

(VI)

umsetzt oder daß man

c) ein Anilin der Formel III mit einem Benzoylurethan der Formel

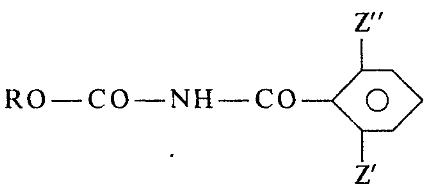

(VII)

umsetzt.

6. Insektizides Mittel, dadurch gekennzeichnet, daß es neben üblichen Hilfs- und/oder Trägerstoffen und gegebenenfalls weiteren Wirkstoffen eine Verbindung nach Anspruch 1 enthält.

7. Verfahren zur Herstellung eines insektiziden Mittels nach Anspruch 6, dadurch gekennzeichnet, daß man eine Verbindung nach Anspruch 1 mit üblichen Hilfs- und/oder Trägerstoffen sowie gegebenenfalls weiteren Wirkstoffen zu Emulsionskonzentraten, Suspensionspulvern, Stäuben, Lösungskonzentraten oder Granulaten verarbeitet.

8. Verwendung von Verbindungen nach Anspruch 1 als pestizide Wirkstoffe.

9. Verbindungen nach Anspruch 1, worin A die Gruppe

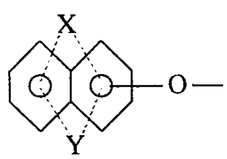

(IIc)

bedeutet.

10. Verbindungen nach Anspruch 1, worin A die Gruppe

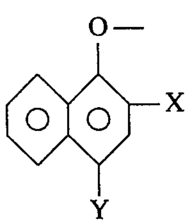

bedeutet, in der einer der Reste X unf Y Chlor, der andere Wasserstoff ist.

**Claims**

1. Compounds of general formula

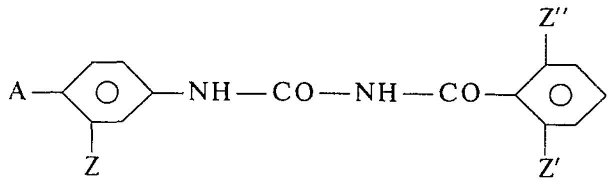

(I)

wherein
A represents one of the groups

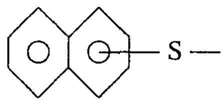 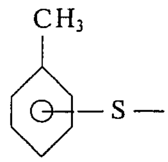 oder 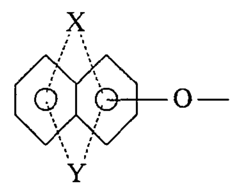

(IIa) (IIb) (IIc)

X and Y, which may be identical or different, represent hydrogen, chlorine or bromine,
Z represents hydrogen or chlorine,
Z' represents chlorine or fluorine, and
Z'' represents hydrogen, chlorine or fluorine.

2. N-[4-($\alpha$-Naphthoxy)-phenyl]-N'-(2-chlorobenzoyl)-urea.
3. N-[4-($\alpha$-Naphthoxy)-phenyl]-N'-(2-chloro-6-fluorobenzoyl)-urea.
4. N-[4-($\beta$-Naphthoxy)-phenyl]-N'-(2-chloro-6-fluorobenzoyl)-urea.
5. Process for the preparation of compounds according to Claim 1, characterised in that

a) an aniline of formula

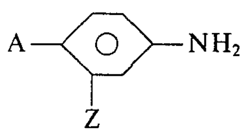

(III)

is reacted with a benzoyl isocyanate of formula

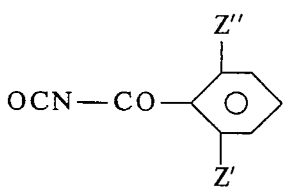

(IV)

or in that

b) a benzamide of formula

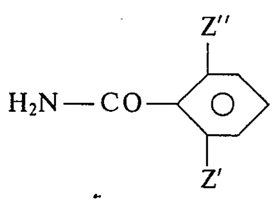

(V)

is reacted with an isocyanate of formula

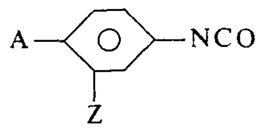 (VI)

or in that

c) an aniline of formula III is reacted with a benzoyl urethane of formula

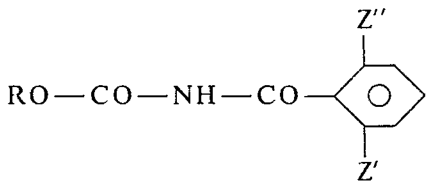 (VIII)

6. Insectidal agent, characterised in that it contains, in addition to conventional excipients and/or carriers and optionally other active substances, a compound according to Claim 1.

7. Process for the preparation of an insecticidal agent according to Claim 6, characterised in that a compound according to Claim 1 is processed with conventional excipients and/or carriers and optionally other active substances to form emulsion concentrates, powders for suspensions, dusting powders, solution concentrates or granulates.

8. Use of compounds according to Claim 1 as pesticidal substances.

9. Compounds according to Claim 1, wherein A represents the group

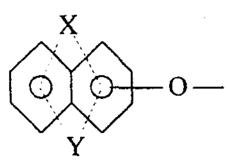 (IIc)

10. Compounds according to Claim 1, wherein A represents the group

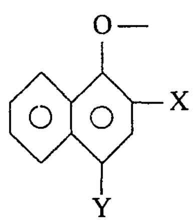

wherein one of the groups X and Y represents chlorine and the other represents hydrogen.

**Revendications**

1. Composés de formule générale

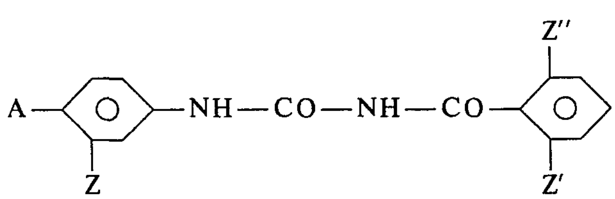 (I)

dans laquelle

A représente l'un des groupes

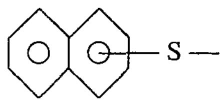 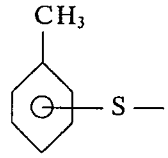 ou 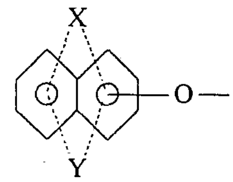

(IIa) (IIb) (IIc)

X et Y, qui peuvent être indentiques ou différents, représentent l'hydrogène, le chlore ou le brome,
Z représente l'hydrogène ou le chlore,
Z' représente le chlore ou le fluor et
Z'' représente l'hydrogène, le chlore ou le fluor.

2. La N-[4-($\alpha$-naphthoxy)-phényl]-N'-(2-chlorobenzoyl)-urée.
3. La N-[4-($\alpha$-naphthoxy)-phényl]-N'-(2-chloro-6-fluorobenzoyl)-urée.
4. La N-[4-($\beta$-naphthoxy)-phényl]-N'-(2-chloro-6-fluorobenzoyl)-urée.
5. Procédé pour la préparation de composés selon la revendication 1, caractérisé en ce que

a) on fait réagir une aniline de formule

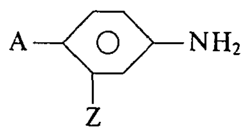 (III)

avec un isocyanate de benzoyle de formule

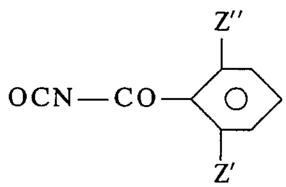 (IV)

ou en ce que

b) on fait réagir un benzamide de formule

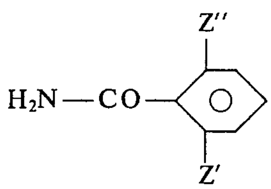 (V)

avec un isocyanate de formule

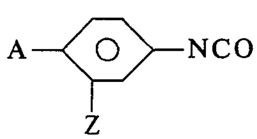 (VI)

ou en ce que

c) on fait réagir une aniline de formule III avec un benzoyl-uréthane de formule

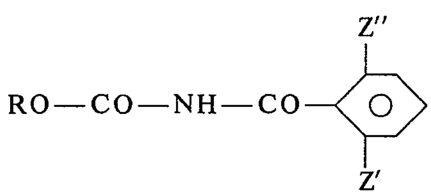 (VIII)

6. Agent insecticide, caractérisé en ce qu'il contient outre des adjuvants et/ou excipients habituels et éventuellement d'autres substances actives un composé selon la revendication 1.

7. Procédé pour la préparation d'un agent insecticide selon la revendication 6, caractérisé en ce qu'on transforme un composé selon la revendication 1 au moyen d'adjuvants et/ou excipients habituels ainsi qu'éventuellement d'autres substances actives en concentrés pour émulsions, poudres pour suspensions, poudres à répandre, concentrés pour solutions ou granulés.

8. Utilisation des composés selon la revendication 1 en tant que substances actives pesticides.

9. Composés selon la revendication 1, où A représente le groupe

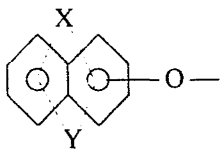

(IIc)

10. Composés selon la revendication 1, où A représente le groupe

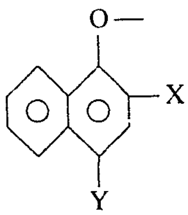

dans lequel l'un des radicaux X et Y est le chlore, l'autre l'hydrogène.